# EUROPEAN PATENT APPLICATION

(11) **EP 1 803 449 A2**
(43) Date of publication of application: **04.07.2007**
(21) Application number: 06025211.1
(22) Date of filing: 06.12.2006
(51) Int. Cl.: A61K 9/50, A61K 33/14, A61K 47/38

(54) **Modified release formulation of lithium carbonate and method for the preparation thereof**

(30) Priority: 22.12.2005 IT BO20050783
(71) Applicant: Vapharma International S.p.A., 61016 Pennabilli (IT)
(72) Inventor: Valducci, Roberto, 47039 Savignano Sul Rubicone (FC) (IT); Alighieri, Tiziano, 47900 Rimini (IT); Avanessian, Serozh, 47900 Rimini (IT)
(74) Representative: Negrini, Elena

(57) **Abstract**

Modified release formulation of lithium carbonate in microgranules or similar comprising, for around 300 Kg of Lithium Carbonate, at least:
- Overall Povidone in quantity between 8.0 Kg and 40.0 Kg;
- Ethylcellulose in quantity between Kg 0. 9 and Kg 3.6.

## Description

### DESCRIPTION OF THE INVENTION

The present invention refers to the pharmacology and particularly refers to a modified release formulation of lithium carbonate and to a method for the preparation of such formulation.

The known formulations of Lithium Carbonate are employed, for instance, as mood stabilizer in the maniac-depressive syndromes and in the cyclotimic depression and in the chronic cluster headache.

The administration of such drugs based on Lithium Carbonate can continue for many years and often for the whole life.

A drawback of some known formulations based on Lithium Carbonate consists in the difficulty to also stabilize the hematic levels of the active principle in the short period, with risks of excessive side effects or insufficient action and with the necessity of constant check of the hematic levels of Lithium Carbonate.

A purpose of the present invention is that to propose a modified release formulation of lithium carbonate able to prolong its absorption and to reduce the variations in the short period of the hematic concentration of the active principle.

Other purpose is to propose a method for the preparation of the modified release formulation of lithium carbonate that is simple, with high yield and able to furnish stable and repetitive results.

The invention foresees that the modified release formulation of lithium carbonate, for instance in microgranules or similar, requires for around 300 Kg of active principle, consisting of Lithium Carbonate, the followings products in the following quantities:

| **Description** | **quantity (Kg)** |
|---|---|
| Overall Povidone | comprised between 8.0 and 40.0 |
| Ethylcellulose | comprised between 0. 9 and 3.6 |
| Talc | comprised between 1.1 and 4.5 |
| Acetone HP Ph. EP | comprised between 8.5 and 34.0 |
| Ethanol BG | comprised between 54. 0 and 220.0 |
| Purified water | comprised between 18.0 and 72.0 |

Particularly the invention foresees that the overall Povidone is reached with a quantity between 3 Kg and 15 Kg of Povidone for the granulation and with a quantity between 5 Kg and 25 Kg of Povidone for application.

The preferred modified release formulation of lithium carbonate requires the followings products in the following quantities and, where specified in the following percentages:

| **Description** | **quantity (Kg)** | **% w/w** |
|---|---|---|
| Lithium Carbonate | around 300 | around 92.45 |
| Povidone granulation | around 7.3 | |
| Povidone application | around 13.16 | |
| **overall% PVP** | | around 6.30 |
| Ethylcellulose | around 1.80 | around 0.55 |
| Talc | around 2.25 | around 0.69 |
| Acetone HP Ph. EP | around 17.01 | |
| Ethanol BG | around 109.1 | |
| Purified water | around 36 | |
| Theoretical yield | around 324.5 | around 100.00 |

Where the preferred quantities in Kg and in% w/w above reported can suffer variations of at least 10%.

At the end of the preparation, the quantities of Acetone HP Ph. EP, Ethanol BG and purified Water in the formulation are virtually void.

It is important to observe that the most greater quantity of Povidone in comparison to the known formulations allows to reduce the quantity of ethylcellulose and to unexpectedly get a noteworthy and advantageous slowing down of the dissolution of the microgranules of the taken Lithium Carbonate nearly maintaining unchanged the percentage of the principle active Lithium Carbonate in the microgranules.

The method for the preparation of the modified release formulation of lithium carbonate foresees the following phases:

### 1. Granulation of the active principle

For the realization of this product 300 Kg of Lithium Carbonate active principle are used, of which 140 Kg are granulated as described as follows:

| Components | Quantity (Kg) |
|---|---|
| Lithium carbonate: | 140 |

| Binding solution: | |
|---|---|
| PVP | 7.3 |
| Water | 30 |
| Water | 6. |

In the granulation phase, the active principle is divided in 2 fractions of 70 Kg.

### 1.1 Preparation of the binding solution

In an inox steel container equipped with pneumatic stirrer 30 Kg of water are introduced, to which 7.3 Kg of PVP are added in small portions; the obtained solution is stirred (for about 18-20 minutes) up to when it results perfectly clear.

### 1.2 Granulation of the 1 st fraction

The granulation is carried out by using a granulator ST 350 in which 70 Kg of Lithium Carbonate are introduced, on which 18.65 Kg of the solution prepared at point 1.1 are sprayed through a membrane pump.

| | |
|---|---|
| Parameters: Speed: | 40 Hz |
| Pressure of spray: | 4 bar |
| Intensity of absorbed current: | 16-19 A |
| Nozzle number: | 6502 |
| Pump capacity: | 888 g/min |
| Duration: | 21 min. |

At the end of the granulation process, the 1st fraction is sieved with a 1000 µm net; the granulate with granulometry lower than 1000 µm is dried for 16 hours in a forced ventilation oven at 60°C. After drying, the product is sieved with a 1200 µm net. The granulate is further sieved with 500 and 840 µm nets.

### 1.3 Granulation of the 2nd fraction

The granulation of the 2nd fraction is carried out as described at point 1.2.

### 1.4 Granulation of the non-conforming granules

The non-conforming granules lower than 500 µm coming from the first 2 fractions are granulated only with water using a granulator ST350 with the same parameters described at point 1.2

### 2. Mixing of the conforming granules

The fractions of conforming granules previously obtained are mixed in coating pan for about 5 minutes to the speed of 2-3 rpm.

At the end of this mixing process, the granulate is discharged by the coating pan and 62 Kg of granules are weighed for the application.

### 3. Micronization for application

### 3.1 Micronization of the active principle

For this phase of micronization the remaining 160 Kg of the starting active principle are withdrawn, introduced in a micronizer Fitz-Mill and micronized with a 120 meshes net.

| | |
|---|---|
| Parameters: Speed of rotation: | 4600 rpm |
| Speed of feeding: | 15 rpm |
| Duration: | 7 hours. |

### 3.2 Micronization of the discards

The non-conforming granules coming from the first two granulations and the granules in excess are introduced in a micronizer Fitz-Mill and micronized with nets of 80 and of 120 meshes.

| | |
|---|---|
| Parameters: Speed of rotation: | 4600 rpm |
| Speed of feeding: | 6 rpm |
| Duration: | 4 hours |

### 4. Application of the active principle

### 4.1 Preparation of the binding solution (PVP 14% in Ethanol)

For the preparation of the binding solution necessary for the phase of application the following components are withdrawn:

| Components | Quantity (Kg) |
|---|---|
| PVP | 13.16 |
| Ethanol BG | 92 |

In an inox steel container equipped with pneumatic stirrer 80 Kg of ethanol are introduced, to which are added in small portions 13.16 Kg of PVP; the obtained solution is stirred (for about 20 minutes) up to complete dissolution.

### 4.2 application of the powder on the starting granules

In an automatic coating pan the 62 Kg of conforming granules obtained at point 2 are introduced, on which the binding solution prepared at point 4.1 is sprayed for 20-30 minutes and the powder obtained by micronization as described at points 3.1 and 3.2 is applied.

**Parameters:**

| | | |
|---|---|---|
| Type of pump: | | membrane |
| Type of gun: | | Wagner GA250AL |
| Nozzle number: | | 827 Gracos |
| Spray interval: | | 5 seconds |
| Cochlea interval: | | 5 seconds from the beginning of use cochlea |
| Cycle of spray: | | for the first 25 minutes at 20", then for 90-180 minutes at 25", then at 30" up to process end |
| Cycle Cochlea: | | 15" for the first 30 minutes, then 20" |
| Speed cochlea: | | 15" for the first 10 minutes, 20" for 5 minutes, 25" up to process end |
| | Temperature air in: | 20-35°C |
| | Temperature product: | to read |
| | Temperature air out: | to read |
| | Sent air (% inverter): | 11 |
| | Depression: | 4 mm H2O |
| | Pressure of spray: | 3 bar |
| | Distance gun nucleuses: | 30 cm |
| | Basket speed: | 12 rpm |

The granules obtained during the application are sieved with a 1340 µm net and dried in forced ventilation oven for about 16 hours at 50°C.

At the end of the drying the granules obtained in the phase of application are further selected at first with a 840 µm net and then with a 1200 µm net with the purpose to separate conforming granules (840-1200 µm) from non-conforming granules.

### 5. Coating of the granules

The conforming granules obtained in the phase of application are divided in 2 equal fractions for the coating with Ethylcellulose.

### 5.1 coating of the 1° sub-batch

For the coating of the 1° sub-batch the following components are withdrawn: Components Quantity (Kg)

| | |
|---|---|
| Ethylcellulose | 0.9 |
| Talc | 1.125 |
| Acetone | 8.55 |
| Ethanol BG | 8.55 |

### Preparation of the 1st retarding solution

In an inox steel container equipped with pneumatic stirrer acetone and ethanol are introduced in the withdrawn quantities (reported in the table) to which ethylcellulose is added in small portions; the obtained solution is stirred (for about 30 minutes) up to complete dissolution.

### Fluid bed coating

The conforming granules of the 1 st fraction are inserted in a fluid bed reactor GPCG 120/200 (with insert Wurster) and on them the solution of ethylcellulose in acetone and ethanol is applied.

| | |
|---|---|
| Parameters: Temperature air in: | 40°C |
| Temperature product: | to read |
| Temperature air out: | to read |
| Shaking interval: | 30" |
| Shaking time: | 10" |
| Pump: | 12 rpm |
| Pressure of nebulisation: | 3 bar |
| Volume: | 2800-3200 m3/hs |
| Pressure diff. product: | to read |
| Pressure diff. filter 1: | to read |
| Pressure diff. filter 2: | to read |
| Partition column: | 55 mm |
| Flap air in: | 52-60% |

The granules coated with this 1 st sub-batch are dried, setting the following parameters:

| | |
|---|---|
| Parameters: Temperature air in: | 60°C |
| Temperature product: | to read |
| Temperature air out: | to read |
| Shaking interval: | 30" |
| Shaking time: | 10" |
| Volume: | 3000 m3/hs |
| Pressure diff. product: | to read |
| Pressure diff. filter 1: | to read |
| Pressure diff. filter 2: | to read |
| Partition column: | 55 mm |
| Flap air in: | 52-60% |

At the end of the drying the obtained granules are selected at first with a 840 µm net and then with a 1340 µm net with the purpose to separate conforming granules (840-1340 µm) from non-conforming granules.

### 5.2 Coating of the 2nd sub-batch

The coating of the 2nd sub-batch is carried out according to the same procedure and using the same parameters described at point 5.1.

The controlled release granules so obtained, having a content of active principle higher than 90%, can be encapsulated or administered through dispensers in the required dosages.

An advantage of the present invention is that to furnish a modified release formulation of lithium carbonate able to prolong its absorption and to reduce the variations in the short period of the hematic concentration of the active principle.

Other advantage is to furnish a method for the preparation of the modified release formulation of lithium carbonate that is simple, with high yield and able to furnish stable and repetitive results.

## Claims

1. Modified release formulation of lithium carbonate in microgranules or similar **characterized in that** it comprises, for around 300 Kg of Lithium Carbonate, at least the following ingredients in the reported quantities:
- Overall Povidone in quantity between 8.0 Kg and 40.0 Kg;
- Ethylcellulose in quantity between Kg 0. 9 and Kg 3.6.

2. Formulation according to claim 1 **characterized in that** it further contains at least a quantity between 1.1 Kg and 4.5 Kg of talc.

3. Formulation according to claims 1 or 2 **characterized in that** the overall quantity of Povidone consists of a quantity between 3 Kg and 15 Kg of Povidone for the granulation and a quantity between 5 Kg and 25 Kg of Povidone for application.

4. Formulation according to claim 1 **characterized in that** it preferably contains for around 300 Kg of Lithium Carbonate, at least:
- Povidone granulation in around 7.3 Kg;
- Povidone application in around 13.16 Kg;
- Ethylcellulose in around 1.80 Kg;
- Talc in around 2.25 Kg;
- Acetone HP Ph. EP in around 17.01 Kg;
- Ethanol BG in around 109.1 kg;
- Purified water in around 36 Kg
for a theoretical yield equal to around 324.5 Kg of formulation.

5. Method for the preparation of the modified release formulation of lithium carbonate of one any of the preceding claims starting from 300 Kg of Lithium Carbonate **characterized in that**:
- to withdraw around 140 Kg of Lithium Carbonate;
- to submit such quantity of Lithium Carbonate to granulation by spraying on it around 37.30 Kg of a first binding solution in a granulator;
- to separate through a 1000 µm net the granulate with granulometry lower to around 1000 µm, to dry that separated granulate and to select the conforming granules with granulometry between 500 µm and 840 µm through respective nets;
- to withdraw around 62 Kg of mixed conforming granules;
- to micronize, through micronizer Fitz-Mill, the remaining 160 Kg of active principle, all the non-conforming granules and those exceeding the 62 Kg of withdrawn conforming granules, obtaining a powder of active principle;
- to submit the 62 Kg of withdrawn conforming granules, to application of the micronized powder of active principle in an automatic coating pan, spraying a binding solution and obtaining some granules on which the micronized powder adheres;
- to sieve with a 1340 µm net, to dry and to select these last conforming and dried granules with granulometry between 840 µm and 1200 µm through respective nets;
- to apply, through a fluid bed reactor, to these last selected granules a retarding solution obtaining coated granules;
- to dry the coated granules and to select those conforming with granulometry between 840 µm and 1340 µm through respective nets;
- to encapsulate or to insert in dispensers the microgranules in the desired dosages of active principle.

6. Method according to claim 5 **characterized by** the fact to divide the 140 Kg of Lithium Carbonate in two fractions of around 70 Kg each, to separately submit such fractions to granulation, separation, drying and to select and to mix, through a coating pan, the conforming granules of the two fractions.

7. Method according to claim 5 **characterized by** the fact to divide the conforming granules on which the micronized powder adheres in two nearly equal sub-batches, to separately submit such sub-batches to application of the solution, to the drying, to the selection and, subsequently to mix the two sub-batches.

8. Method according to claim 5 **characterized by** the fact to realize the first binding solution in an inox steel container equipped with pneumatic stirrer, introducing in it 30 Kg of water, to which are added in small portions 7.3 Kg of PVP and stirring for about 18-20 minutes such solution up to when it results perfectly clear.

9. Method according to claim 5 **characterized by** the fact to realize the second binding solution in an inox steel container equipped with pneumatic stirrer, introducing in it 80 Kg of ethanol to which are added in small portions 13.16 Kg of PVP and stirring for about 20 minutes the obtained solution up to complete dissolution.

10. Method according to claim 5 **characterized by** the fact to realize the second solution introducing, in an inox steel container equipped with pneumatic stirrer, around 8.55 Kg of acetone and around 8.55 Kg of ethanol to which 0.9 Kg of ethylcellulose are added in small portions and to stir the solution for about 30 minutes up to complete dissolution.

11. Method according to claim 6 **characterized by** the fact to realize the granulation using a granulator St 350 in which 70 Kg of Lithium Carbonate are introduced, on which 18.65 Kg of the first solution are sprayed through a membrane pump using the following parameters: speed 40 Hz, pressure of spray 4 bar, intensity of absorbed current 16-19 Á., Nozzle n. 6502, pump capacity 888 g/min, duration 21 min.

12. Method according to claim 5 **characterized by** the fact of micronizing the starting 160 Kg of active principle through the micronizer Fitz-Mill with a 120 meshes net using the following parameters: speed of rotation 4600 rpm, speed of feeding 15 rpm, duration 7 hours; and of micronizing the non-conforming granules and the granules in excess through the micronizer Fitz-Mill with 80 and 120 meshes nets using the following parameters: speed of rotation 4600 rpm, speed of feeding 6 rpm duration 4 hours.

13. Method according to claim 5 **characterized by** the fact to apply the powder of active principle on the 62 Kg of conforming granules spraying the second binding solution for 20-30 minutes in the automatic coating pan using the following parameters: type of pump membrane, type of gun Wagner GA250AL, Nozzle n. 827 Gracos, spray interval 5 seconds, cochlea interval 5 seconds from the beginning of cochlea use, cycle of spray for the first 25 minutes at 20", then for 90-180 minutes at 25", then at 30" up to process end, cycle cochlea 15" for the first 30 minutes, then at 20", cochlea speed 15" for the first 10 minutes, 20" for 5 minutes, 25" up to process end, temperature air in 20-35°C, temperature product to read, temperature air out to read, sent air (% inverter) 11, depression 4 mm H2O, pressure of spray 3 bar, distance gun nucleuses 30 cm, basket speed 12 rpm.

14. Method according to claim 5 **characterized by** the fact to realize the bed fluid coating of the conforming granules through the fluid bed reactor GPCG 120/200 (with insert Wurster) applying on them the solution of ethylcellulose in acetone and ethanol using the following parameters: temperature air in 40°C, temperature product to read, temperature air out to read, shaking interval 30", shaking time 10", pump 12 rpm, pressure of nebulisation 3 bar, volume 2800-3200 m3/h, pressure diff. product to read, pressure diff. filter 1 to read, pressure diff. filter 2 to read, partition column 55 mm, flap air in 52-60%.

15. Method according to claim 5 **characterized by** the fact to dry the coated granules using the following parameters: Temperature air in 60°C, temperature product to read, temperature air out to read, shaking interval 30", shaking time 10", volume 3000 m3/h, pressure diff. Product to read, pressure diff. filter 1 to read, pressure diff. filter 2 to read, partition column 55 mm, flap air in 52-60%.
